# EUROPEAN PATENT SPECIFICATION

(11) **EP 2 928 420 B1**
(45) Date of publication and mention of the grant of the patent: **28.03.2018**
(21) Application number: 13824228.4
(22) Date of filing: 04.12.2013
(51) Int. Cl.: A61F 5/00, A61F 2/04, A61B 17/12

(54) **INFLATABLE OBSTRUCTION DEVICE FOR THE PYLORIC SPHINCTER**
AUFBLASBARE OBSTRUKTIONSVORRICHTUNG FÜR DEN MAGENPFÖRTNERMUSKEL
DISPOSITIF D'OBSTRUCTION GONFLABLE POUR LE SPHINCTER PYLORIQUE

(30) Priority: 05.12.2012 US 201213705359; 14.04.2013 US 201313862429
(43) Date of publication of application: 14.10.2015
(73) Proprietor: Easynotes Ltd., 73150 Kfar Truman (IL)
(72) Inventor: KLEIN, David, 76209 Rehovot (IL); FABIAN, Izhak, 73150 Kfar Truman (IL); ALTMAN, Nir, 90912 Kfar Etzion (IL); HAAS, Steven, 44864 Kochav Yair (IL); HIRSCH, Yoav, 71720 Modiin (IL); MENDELEWICZ, Ran, 46321 Herzlia (IL)
(74) Representative: Jaeger, Michael David
(86) International application number: PCT/US2013/072943
(87) International publication number: WO 2014/089129

(56) References cited:
- WO-A1-2008/106041
- WO-A2-2005/009288
- US-A- 3 411 506
- US-A1- 2005 273 060

## Description

### FIELD OF THE INVENTION

The present invention generally relates to devices for obstructing or reducing flow through a body lumen, in particular for obstructing or reducing flow of gastric contents across the pyloric valve, and a delivery system capable of inflating and deflating balloons of the device.

### BACKGROUND OF THE INVENTION

In the prior art, when an occlusion of the pylorus is required in the course of a gastroplasty procedure or in a procedure that involves the duodenum, the surgeon staples the pylorus shut (in the stomach) and this is a short term occlusion to allow the duodenum to recover from an operation. Transpyloric devices have also been proposed, which may partially and/or intermittently obstruct the pylorus, thereby decreasing the flow of gastric contents into the duodenum.

However, there are some chronic patients who require long-term obstruction of the pylorus. Long-term obstruction is problematic. The gastrointestinal (GI) environment applies forces that tend to move the plug out of place over time, including normal peristaltic movement and other movements, such as coughing or vomiting. The plug must withstand the chemical environment, too. Accordingly, a chronic pyloric plug must be fixed stronger around the pylorus and have a tighter fit than temporary solutions. A gastrointestinal apparatus with proximal and distal expandable chambers is disclosed by US 2005/273060 A1. Two separate inflation ports arranged at the proximal end of the device can be used to selectively inflate either of the balloons.

### SUMMARY OF THE INVENTION

The present invention seeks to provide an obstruction device (also called pyloric plug or just "plug") for obstructing or reducing flow through a body lumen, in particular for obstructing or reducing flow of gastric contents across the pyloric valve (pylorus), as is described more in detail hereinbelow. The device is particularly useful in a transoral gastrointestinal procedure, but the invention is not limited to transoral gastroplasty, and may be used in other laparoscopic, endoscopic, or natural orifice procedures in other body lumens. The plug is designed to be fully operative over a long time, such as but not limited to, between six months and many years. The device can be removed, if desired, and can also be re-implanted.

The plug includes two balloons, one proximal and the other distal, mounted on a shaft. The proximal obstruction balloon is arranged to fit in the stomach, whereas the distal obstruction balloon is arranged to fit in the duodenum. When inflated, both balloons expand towards the pylorus from opposite sides on the pylorus, thus fixing the plug in place.

The plug is particularly useful to stop the flow of stomach contents to the proximal gut which includes the duodenum and the initial part of the jejunum. Such a need arises, for example, after creating an alternative path of flow through a gastro-jejunum anastomosis which bypasses the proximal gut. There could be other cases when this need arises, such as after surgery in the duodenum area or in the pancreas or bile outputs to the duodenum. Another indication could be the need to operate endoscopically on the stomach with an inflated stomach. In this case, the plug keeps the inflating air in the stomach and it does not bloat the intestine.

The plug can be used in a method for creating an anastomosis between a stomach and a portion of a small intestine, wherein the plug is used to control passage of stomach contents through the pylorus during and after creation of the anastomosis. For example, before the anastomosis has been created, the plug would allow passage of material therethrough, but after creation of the anastomosis the pylorus plug would either completely block flow (so that material only flows through the anastomosis) or partially block flow (so that material can flow through both the plug and the anastomosis).

The present invention also seeks to provide a delivery system for use with obstruction devices (plugs) that have more than one obstruction balloons. As is described more in detail hereinbelow, the delivery system is capable of inserting the obstruction device, retrieving it, and inflating and deflating balloons of the obstruction device.

There is thus provided in accordance with an embodiment of the present invention an assembly including an obstruction device including a proximal obstruction balloon and a distal obstruction balloon mounted on a shaft, the balloons being inflatable via an inflation lumen, and a delivery system that includes an insertion tool and an injection site assembly assembled with one of the balloons, the insertion tool including a connector which is connectable to the injection site assembly and which permits passing tools and injection fluid therethrough.

### BRIEF DESCRIPTION OF THE DRAWINGS

The present invention will be understood and appreciated more fully from the following detailed description taken in conjunction with the drawings in which:
Fig. 1 is a simplified illustration of an obstruction device and delivery system, constructed and operative in accordance with an embodiment of the present invention;
Figs. 2A and 2B are simplified illustrations of an insertion tool of the delivery system, respectively during and after coupling with the obstruction device, in accordance with an embodiment of the present invention;
Figs. 3A and 3B are simplified illustrations of using the delivery system to inflate distal and proximal balloons, respectively, of the obstruction device, in accordance with an embodiment of the present invention;
Fig. 4 is a simplified illustration of a deflation mechanism of the delivery system, in accordance with an embodiment of the present invention;
Figs. 5A and 5B are simplified perspective and sectional illustrations, respectively, of an obstruction device with inflatable distal and proximal balloons, constructed and operative in accordance with another embodiment of the present invention, and having an off-center puncturable proximal deflator balloon in fluid communication with the distal balloon for deflating the distal balloon;
Figs. 6A and 6B are simplified perspective and sectional illustrations, respectively, of an obstruction device with inflatable distal and proximal balloons, constructed and operative in accordance with yet another embodiment of the present invention, and having a central puncturable proximal deflator balloon in fluid communication with the distal balloon for deflating the distal balloon; and
Fig. 7A is a simplified illustration of a delivery system for any of the obstruction devices of the invention, constructed and operative in accordance with another embodiment of the present invention, wherein the delivery system includes a proximal handle and a distal insertion tool that has an inflation tube mounted on a shaft for relatively large movement and relatively fine adjustment movement; and
Figs. 7B and 7C are illustrations of the delivery system of Fig. 7A, respectively before and after advancing the inflation tube distally.

### DETAILED DESCRIPTION OF EMBODIMENTS

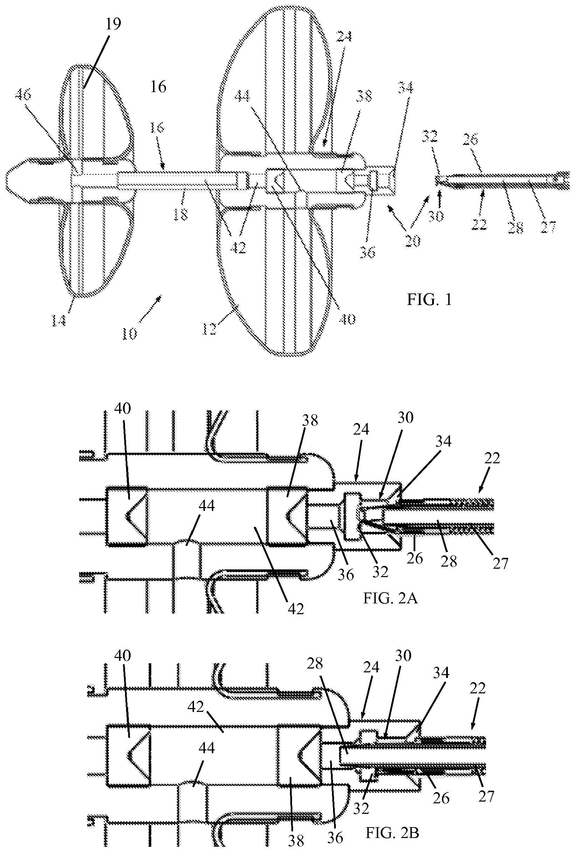

Reference is now made to Fig. 1, which illustrates an obstruction device (or plug) 10 and delivery system 20, constructed and operative in accordance with an embodiment of the present invention.

Obstruction device 10 includes a proximal obstruction balloon 12 and a distal obstruction balloon 14 mounted on a shaft 16. A portion of shaft 16, referred to as neck 18 or neck portion 18, provides a gap between proximal balloon 12 and distal balloon 14. Neck 18 can have different lengths and thicknesses depending on the application; for example, the dimensions of neck 18 are correlated to the usual width of the pylorus muscle.

The proximal obstruction balloon 12 is arranged to fit in the stomach, whereas the distal obstruction balloon 14 is arranged to fit in the duodenum. When inflated, balloons 12 and 14 expand towards the pylorus from opposite sides on the pylorus, thus fixing the plug 10 in place.

In accordance with an alternative embodiment of the present invention, distal obstruction balloon 14 includes a plurality of internal or external anchoring arms 19. In one embodiment, anchoring arms 19 are constructed from folds in balloon 14. One purpose of arms 19 is to help anchor the device against the pylorus in the duodenum. Another purpose is to create a non-uniform surface for pushing against tissue (e.g., the distal side of the pylorus). The non-uniform surface may help prevent creating constant pressure against the duodenal side of the pylorus; constant pressure has the disadvantageous risk of causing a sore, like a pressure sore, on the tissue

The anchoring arms 19 may be useful for maintaining the balloon 14 in place in the duodenum, because they can maintain anchoring forces on the duodenum walls even in the presence of variable pressure on the duodenum walls. Since there is generally less abrasion on the distal side of the pylorus, a perfect seal is not necessary, and anchoring is more important than sealing. On the stomach side, balloon 12 may have a uniform inflated shape that blocks flow from the stomach. Alternatively, balloon 12 may also be provided with anchoring arms.

In another embodiment, anchoring arms 19 may be arcuate loops of a flexible yet strong material suitable for anchoring against the intestinal walls, such as but not limited to, NITINOL or stainless steel alloy.

Delivery system 20 includes an insertion tool 22 (separate from the plug) and an injection site assembly 24 assembled with one of the balloons, preferably, but not necessarily, the proximal balloon 12. Insertion tool 22 includes a shaft 26 that has a hollow lumen 27 for passing therethrough an inflation tube 28 (tube, catheter or syringe and the like). The distal end of shaft 26 is provided with a connector 30, which connects to injection site assembly 24 and which permits passing injection tools, injection fluid and other tools or substances therethrough.

In one embodiment, connector 30 includes a plurality of resilient fingers 32 (made of a suitable resilient, medically safe material, such as but not limited to, stainless steel, NITINOL or others) which serve as leaf springs.

In one embodiment, injection site assembly 24 includes a proximal insertion port 34 and a distal receiving member 36, wherein the proximal insertion port 34 is of smaller diameter than the distal receiving member 36. Injection site assembly 24 further includes a proximal septum 38, which serves as the proximal injection site 38, and a distal septum 40, which serves as the distal injection site 40, which is axially spaced from the proximal injection site 38. An inflation lumen 42 extends through shaft 16 and is in fluid communication with one or more proximal inflation ports 44 for inflation of proximal balloon 12 and with one or more distal inflation ports 46 for inflation of distal balloon 14.

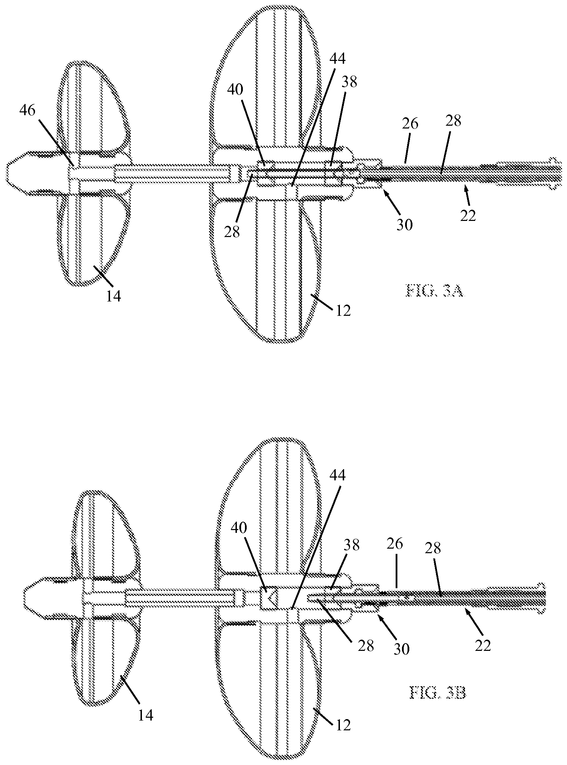

Reference is now made to Figs. 2A and 2B, which illustrate connecting insertion tool 22 with injection site assembly 24. In Fig. 2A, resilient fingers 32 of connector 30 are inserted into and through the proximal insertion port 34. After passing through the relatively narrow proximal insertion port 34, fingers 32 can expand outwards into the space of distal receiving member 36. As seen in Fig. 2B, inflation tube 28 is inserted through lumen 27 of shaft 26 and presses fingers 32 against the inner surfaces of distal receiving member 36 (which are complementarily shaped in accordance with the shape of fingers 32). Inflation tube 28 can now be advanced distally through proximal injection site 38, as is now explained with reference to Figs. 3A and 3B.

In Fig. 3A, inflation tube 28 has advanced distally through both proximal injection site 38 and distal injection site 40. Distal balloon 14 is inflated with saline, air or other fluid, from a fluid source (not shown) flowing through distal inflation port 46. In Fig. 3B, inflation tube 28 is withdrawn proximally so that distal septum 40 is now sealed and proximal balloon 12 is inflated with fluid flowing through proximal inflation port 44. Optionally, proximal balloon 12 could be inflated first. Each balloon expands in a required direction so that as it expands, it increases pressure on the pylorus.

Both balloons may be deflated by connecting tube 28 to a source of negative pressure (vacuum) and sequentially introducing tube 28 to each injection site; instead of injecting fluid to the balloon, the balloon is emptied by suction. Deflation may instead be done by a deflation mechanism, as is now explained.

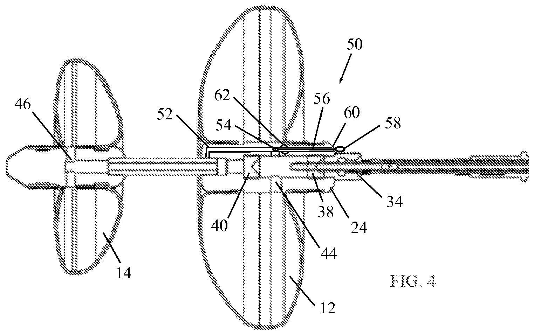

Reference is now made to Fig. 4, which illustrates a deflation mechanism 50 of the delivery system, in accordance with an embodiment of the present invention.

In this embodiment, injection site assembly 24 is formed with a deflation lumen 52 which is in fluid communication with distal inflation port 46, on the distal side of distal septum 40, and with proximal inflation port 44, on the distal side of proximal septum 38. A stopper 54 (e.g., an elastomeric ball) is mounted on a distal end of a slender member 56, which may be provided with a proximal grasping member 58. The slender member 56 is introduced through an outer opening 60 of deflation lumen 52. The slender member 56 may be manipulated with a grasping tool (not shown).

As long as stopper 54 is sealingly seated in proximal inflation port 44, stopper 54 blocks and seals fluid from leaking out of both balloons 12 and 14. When stopper 54 is moved away from proximal inflation port 44, such as to an enlargement 62 in lumen 52, stopper 54 no longer seals the balloons; both balloons 12 and 14 become deflated due to fluid flowing out of them through deflation lumen 52.

In the illustrated embodiment, deflation mechanism 50 deflates both balloons simultaneously. Alternatively, deflation mechanism 50 can be constructed to deflate the balloons one at a time.

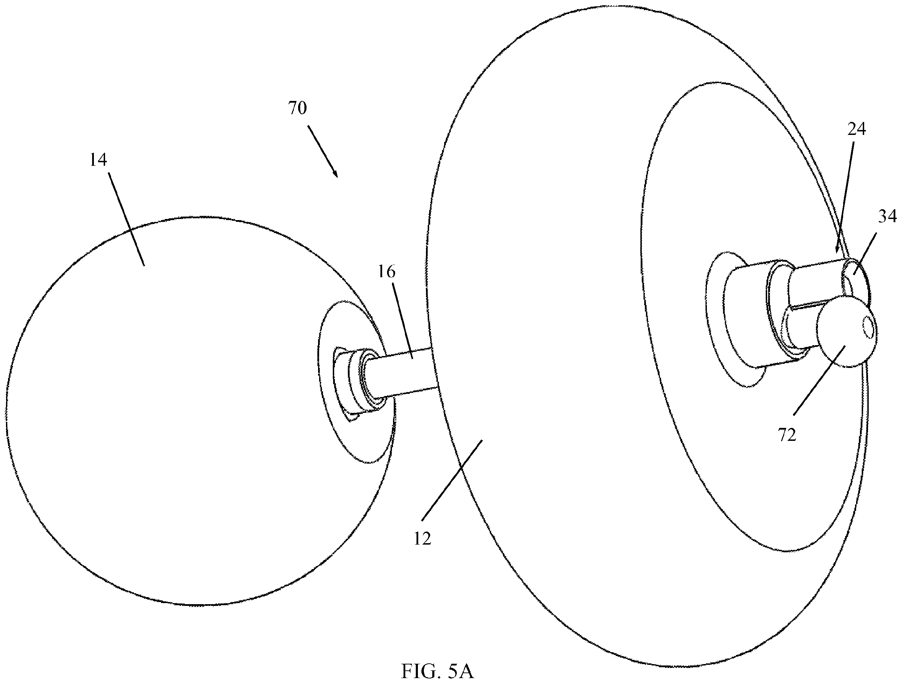

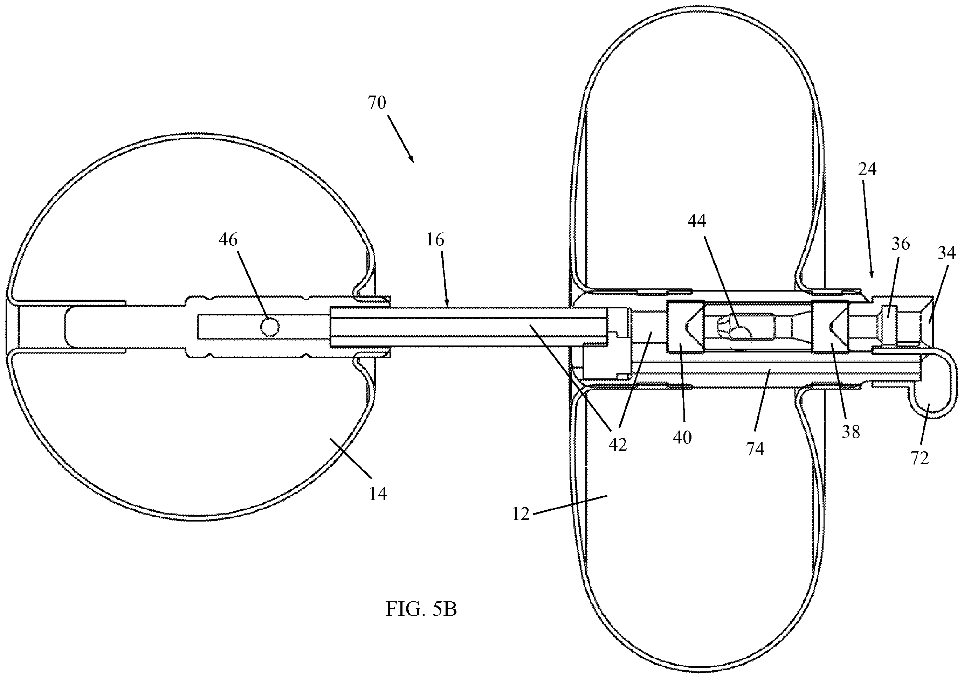

Reference is now made to Figs. 5A and 5B, which illustrate an obstruction device 70, constructed and operative in accordance with another embodiment of the present invention. Device 70 is similar in construction to device 10, with like numerals denoting like elements. Device 70 differs from device 10 in that it includes a different deflation mechanism, as is now explained.

Device 70 includes an off-center puncturable proximal deflator balloon 72, which is in fluid communication with the distal balloon 14 via a lumen 74 which is in fluid communication with inflation lumen 42 (Fig. 5B). When distal balloon 14 is inflated, deflator balloon 72 also inflates. When it is desired to deflate distal balloon 14, one simply punctures deflator balloon 72 with a sharp instrument (not shown). Deflator balloon 72 is easily accessible on account of its being proximal to proximal balloon 12. It is noted that deflator balloon 72 is much smaller in size than distal balloon 14. This ensures that puncturing deflator balloon 72 will cause the fluid to be expelled from distal balloon 14. If deflator balloon 72 were similar in size as or bigger than distal balloon 14, the pressure difference between deflator balloon 72 and distal balloon upon puncturing deflator balloon 72 could be such that fluid would be expelled from deflator balloon 72 towards distal balloon 14, with the result that distal balloon 14 would remain inflated and not diminish in size.

It is noted that distal balloon 14 may have a distal inverted neck (Fig. 5B) so that no tube protrudes distally of the balloon 14; this ensures a smooth distal end even after inflation.

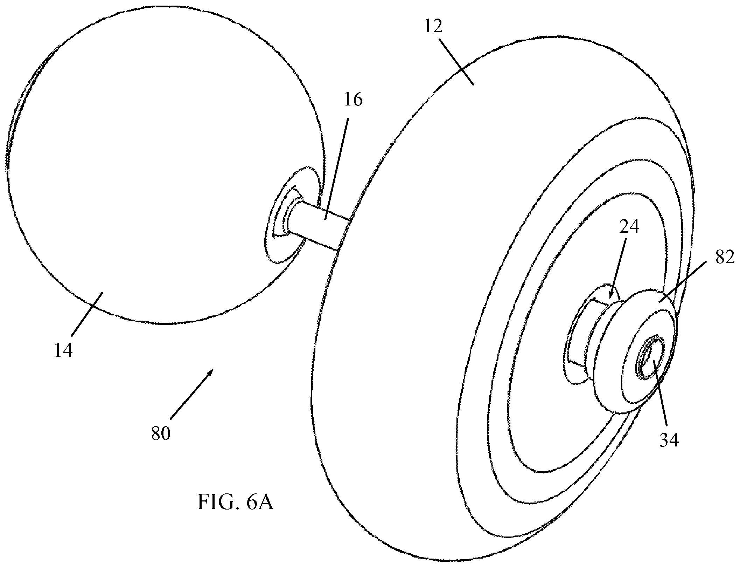

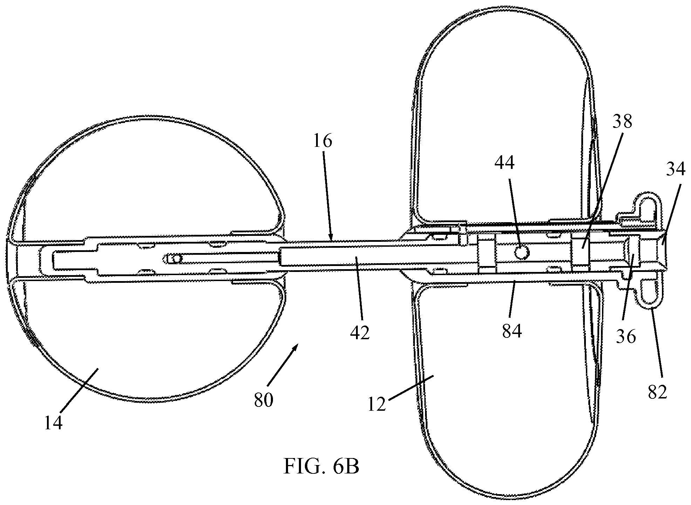

Reference is now made to Figs. 6A and 6B, which illustrate an obstruction device 80, constructed and operative in accordance with yet another embodiment of the present invention. Device 80 is similar in construction to device 70, with like numerals denoting like elements. Device 80 differs from device 70 in that device 80 has a deflator balloon 82 which is central (coaxial with balloons 12 and 14), instead of being off-center as deflator balloon 72 of Figs. 5A and 5B. As similarly described for device 70, deflator balloon 82 is in fluid communication with the distal balloon 14 via a lumen 84 which is in fluid communication with inflation lumen 42 (Fig. 6B).

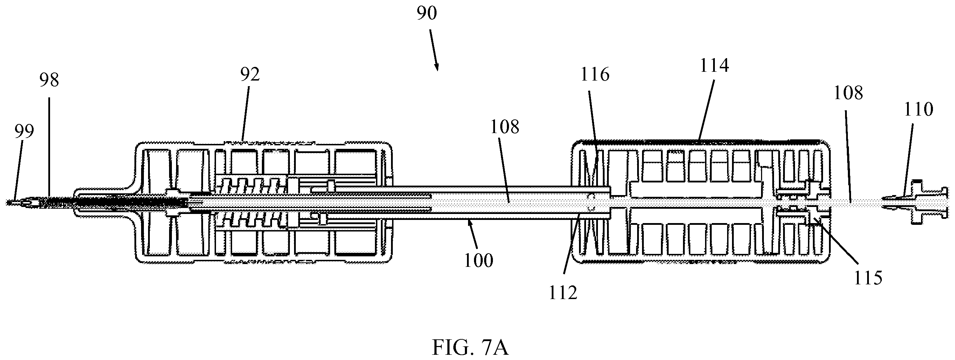

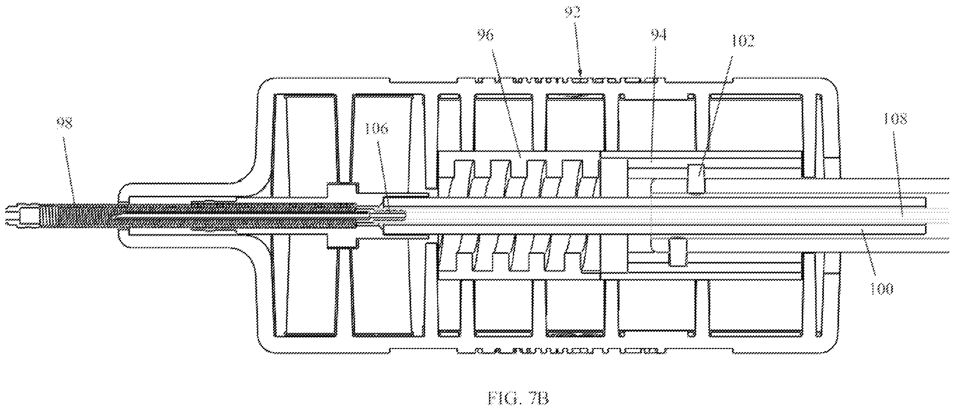

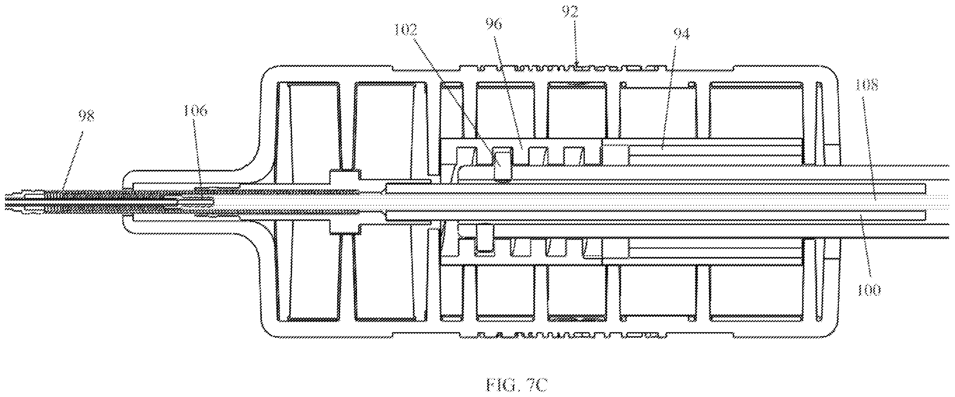

Reference is now made to Fig. 7A, which illustrates a delivery system 90 for any of the obstruction devices of the invention, constructed and operative in accordance with another embodiment of the present invention. Delivery system 90 includes a distal insertion tool 92 that has a mechanism for relatively large movement (coarse adjustment) and relatively fine movement (fine adjustment) of a coil shaft 98 (seen best in Figs. 7B and 7C). The coil shaft 98 has a similar purpose to the shaft 26 described above: coil shaft 98 is hollow for passing therethrough a tube 108 (similar to tube 28 described above, which may be a tube, catheter or syringe and the like), and the distal end of coil shaft 98 is provided with a connector 99 with resilient fingers, which connects to the injection site assembly and permits passing injection tools, injection fluid and other tools or substances therethrough (as described above).

Coil shaft 98 is mounted on the distal end of a shaft 100 which is mounted for rotation and translation in distal insertion tool 92. Shaft 100 has a threaded portion 102 (such as male threads 102) that can slide freely without threaded engagement in a bore 94 formed in a proximal portion of distal insertion tool 92. This sliding, non-threaded movement is the coarse adjustment portion of the mechanism. The threaded portion 102, upon sufficient distal movement in bore 94, will threadingly engage a distal threaded bore 96 formed in distal insertion tool 92. The threaded engagement of threaded portion 102 with threaded bore 96 is the fine adjustment portion of the mechanism.

The coil shaft 98 has a proximal fluid connector 106 for mating with tube 108 that leads from a fluid source device 110 (Fig, 7A), such as, but not limited to, a syringe. Tube 108 is used to inflate the balloons with fluid at the inflation ports, as described above. A proximal end 112 of shaft 100 is mounted in a proximal handle 114. Proximal handle 114 has a proximal bearing 115 for accepting and supporting tube 108 and also may have an actuator 116 (such as, but not limited to, a motor) for connecting to and rotating shaft 100. Rotation of shaft 100 causes distal or proximal movement of coil shaft 98 and tube 108. Bearing 115 ensures that tube 108 does not twist during rotation and linear motion of tube 108. Thus, the system can be used for coarse movement and fine adjustment of coil shaft 98 and tube 108 so that coil shaft 98 and tube 108 can be precisely positioned at the different injection sites described above for inflation or deflation of the proximal and distal balloons.

## Claims

1. An assembly comprising:
an obstruction device (10) comprising a proximal obstruction balloon (12) and a distal obstruction balloon (14) mounted on a shaft (16), said balloons being inflatable via an inflation lumen (42),
an injection site assembly (24) assembled with one of said balloons, and
a delivery system (20) that comprises an insertion tool (22) comprising a connector (30) which is connectable to said injection site assembly (24) and which permits passing tools and injection fluid therethrough,
wherein said injection site assembly (24) comprises a proximal septum (38), which serves as a proximal injection site, and a distal septum (40), which serves as a distal injection site and which is axially spaced from said proximal injection site.

2. The assembly according to claim 1, wherein said insertion tool (22) comprises a shaft (26) that has a hollow lumen (27) for passing therethrough an inflation tube (28).

3. The assembly according to claim 1, wherein said connector (30) comprises a plurality of resilient fingers (32) insertable into said injection site assembly (24).

4. The assembly according to claim 1, wherein said injection site assembly (24) comprises an insertion port (34) and a receiving member (36) distal to said insertion port (34).

5. The assembly according to claim 1, wherein said inflation lumen (42) extends through said shaft (16) of said obstruction device (10) and is in fluid communication with one or more proximal inflation ports (44) for inflation of said proximal balloon (12) and with one or more distal inflation ports (46) for inflation of said distal balloon (14).

6. The assembly according to claim 2, wherein said connector (30) comprises a plurality of resilient fingers (32) insertable into said injection site assembly (24), and said inflation tube (28) is arranged to pass through said hollow lumen (27) and press said fingers (32) against inner surfaces of said injection site assembly (24).

7. The obstruction device (10) according to claim 1, wherein a neck portion (18) of said shaft (16) comprises a gap between said proximal obstruction balloon (12) and said distal obstruction balloon (14).

8. The obstruction device (10) according to claim 1, further comprising a deflation mechanism (50) operative to deflate said balloons.

9. The obstruction device (10) according to claim 8, wherein said inflation lumen (42) is in fluid communication with one or more proximal inflation ports (44) for inflation of said proximal balloon (12) and with one or more distal inflation ports (46) for inflation of said distal balloon (14), and wherein said deflation mechanism (50) comprises a deflation lumen (52) formed in said injection site assembly (24), which is in fluid communication with said distal inflation port (46) and with said proximal inflation port (44), and wherein a stopper (54) is arranged to be sealingly seated in said proximal inflation port (44) to seal fluid from leaking out of said balloons and to be moved away from said proximal inflation port (44) to permit evacuating fluid from said balloons.

10. The obstruction device (70) according to claim 8, wherein said deflation mechanism comprises a puncturable proximal deflator balloon (72) in fluid communication with said distal balloon (14).

11. The obstruction device (70) according to claim 10, wherein said deflator balloon (72) is smaller in size than said distal balloon (14).

12. The obstruction device (70) according to claim 10, wherein said delivery system (90) comprises a distal insertion tool (92) that has a shaft (100) operative to cause coarse and fine movement of said inflation tube (28).

## Patentansprüche

1. Anordnung, die aufweist:
eine Obstruktionsvorrichtung (10) mit einem proximalen Obstruktionsballon (12) und einem distalen Obstruktionsballon (14), wobei die Ballons auf einer Welle (16) sitzen und über ein Aufweitlumen (42) aufweitbar sind;
eine Injektionsortanordnung (24), die mit einem der Ballons zusammengefügt ist; und
ein Ausgabesystem (20), das ein Einsetzwerkzeug (22) mit einem Verbinder (30) aufweist, der mit der Injektionsortanordnung (24) verbindbar ist und den Durchgang von Werkzeugen und Injektionsfluid erlaubt;
wobei die Injektionsortanordnung (24) ein proximales Septum (38), das als proximaler Injektionsort dient, und ein distales Septum (40) aufweist, das als distaler Injektionsort dient und vom proximalen Injektionsort axial beabstandet ist.

2. Anordnung nach Anspruch 1, bei der das Werkzeug (22) einen Schaft (26) aufweist, der ein hohles Lumen (27) für den Durchgang eines Aufweitschlauchs (28) enthält.

3. Anordnung nach Anspruch 1, bei der der Verbinder (30) eine Vielzahl federelastischer Finger (32) aufweist, die in die Injektionsortanordnung (24) einsetzbar sind.

4. Anordnung nach Anspruch 1, bei der die Injektionsortanordnung (24) eine Einführöffnung (34) und distal zu dieser ein Aufnahmeglied (36) aufweist.

5. Anordnung nach Anspruch 1, bei der das Aufweitlumen (26) durch die Welle (16) der Obstruktioneinrichtung (10) verläuft und zum Aufweiten des proximalen Ballons (12) und des proximalen Ballons (14) in Strömungsverbindung mit einer oder mehr der Aufweitöffnungen (44 bzw. 46) steht.

6. Anordnung nach Anspruch 2, bei der der Verbinder (30) eine Vielzahl federelastischer Finger (32) aufweist, die in die Injektionsortanordnung (24) einführbar sind, und der Aufweitschlauch (28) angeordnet ist, durch das hohle Lumen (27) zu verlaufen und die Finger (32) auf die Innenflächen der Injektionsortanordnung (2) zu drücken.

7. Obstruktionsanordnung (10) nach Anspruch 1, bei der der Halsabschnitt (18) der Welle (16) einen Spalt zwischen dem proximalen und dem distalen Obstruktionsballon (12 bzw. 14) aufweist.

8. Obstruktionseinrichtung (10) nach Anspruch 1, weiterhin mit einer Aufweitmechanik (50), mit der die Ballons betrieblich druckentlastbar sind.

9. Obstruktionseinrichtung (10) nach Anspruch 8, bei der das Aufweitlumen (42) zum Aufweiten des proximalen Ballons (12) mit einem oder mehr proximalen Aufweitöffnungen (44) und zum Aufweiten des distalen Ballons (14) mit einer oder mehr distalen Aufweitöffnungen (46) in Strömungsverbindung steht, bei der weiterhin die Aufweitmechanik (50) ein Aufweitlumen (52) aufweist, das in der Injektionsortanordnung (24) ausgebildet ist und in Strömungsverbindung mit der distalen und der proximalen Aufweitöffnung (44 bzw. 46) steht, und bei der ein Verschlussstopfen (54) dicht abschließend in die proximale Aufweitöffnung (44) einsetzbar ist, um Fluid gegen ein Entweichen aus den Ballons abzudichten, und aus der proximalen Aufweitöffnung (44) lösbar ist, um ein Entfernen von Fluid aus den Ballons zu erlauben.

10. Obstruktionseinrichtung (70) nach Anspruch 8, bei der die Aufweitmechanik einen punktierbaren proximalen Aufweitballon (72) in Strömungsverbindung mit dem distalen Ballon (14) aufweist.

11. Obstruktionseinrichtung (70) nach Anspruch 10, bei der der Aufweitballon (72) größenmäßig kleiner ist als der distale Ballon (14).

12. Obstruktionseinrichtung (70) nach Anspruch 10, bei der die Ausgabeeinrichtung (90) ein distales Einführwerkzeug (92) mit einem Schaft (100) aufweist, mit dem betrieblich Grob- und Feinbewegungen des Aufweitschlauchs verursachbar sind.

## Revendications

1. Ensemble comprenant :
un dispositif d'obstruction (10) comprenant un ballonnet d'obstruction proximal (12) et un ballonnet d'obstruction distal (14) montés sur un arbre (16), lesdits ballonnets pouvant être gonflés par l'intermédiaire d'une lumière de gonflage (42),
un ensemble formant site d'injection (24) assemblé avec l'un desdits ballonnets, et
un dispositif d'administration (20) qui comprend un outil d'insertion (22) comprenant un raccord (30) qui peut être raccordé audit ensemble formant site d'injection (24) et qui permet le passage à travers d'outils et d'un fluide d'injection,
dans lequel ledit ensemble formant site d'injection (24) comprend une membrane proximale (38), qui sert de site d'injection proximal, et une membrane distale (40), qui sert de site d'injection distal et qui est espacé axialement par rapport audit site d'injection proximal.

2. Ensemble selon la revendication 1, dans lequel ledit outil d'insertion (22) comprend un arbre (26) qui comporte une lumière creuse (27) destinée à faire passer un tube de gonflage (28) à travers.

3. Ensemble selon la revendication 1, dans lequel ledit raccord (30) comprend une pluralité de doigts élastiques (32) pouvant être insérés dans ledit ensemble formant site d'injection (24).

4. Ensemble selon la revendication 1, dans lequel ledit ensemble formant site d'injection (24) comprend un orifice d'insertion (34) et un élément de réception (36) distal par rapport audit orifice d'insertion (34).

5. Ensemble selon la revendication 1, dans lequel ladite lumière de gonflage (42) s'étend à travers ledit arbre (16) dudit dispositif d'obstruction (10) et est en communication fluidique avec un ou plusieurs orifices de gonflage proximaux (44) afin de gonfler ledit ballonnet proximal (12) et avec un ou plusieurs orifices de gonflage distaux (46) afin de gonfler ledit ballonnet distal (14).

6. Ensemble selon la revendication 2, dans lequel ledit raccord (30) comprend une pluralité de doigts élastiques (32) pouvant être insérés dans ledit ensemble formant site d'injection (24), et ledit tube de gonflage (28) est agencé de manière à passer à travers ladite lumière creuse (27) et presser lesdits doigts (32) contre des surfaces internes dudit ensemble formant site d'injection (24).

7. Dispositif d'obstruction (10) selon la revendication 1, dans lequel une partie de col (18) dudit arbre (16) comprend un jeu entre ledit ballonnet d'obstruction proximal (12) et ledit ballonnet d'obstruction distal (14).

8. Dispositif d'obstruction (10) selon la revendication 1, comprenant, en outre, un mécanisme de dégonflage (50) pouvant servir à dégonfler lesdits ballonnets.

9. Dispositif d'obstruction (10) selon la revendication 8, dans lequel ladite lumière de gonflage (42) est en communication fluidique avec un ou plusieurs orifices de gonflage proximaux (44) afin de gonfler ledit ballonnet proximal (12) et avec un ou plusieurs orifices de gonflage distaux (46) afin de gonfler ledit ballonnet distal (14), et dans lequel ledit mécanisme de dégonflage (50) comprend une lumière de dégonflage (52) formée sur ledit ensemble formant site d'injection (24), qui est en communication fluidique avec ledit orifice de gonflage distal (46) et avec ledit orifice de gonflage proximal (44), et dans lequel un bouchon (54) est agencé afin de pouvoir être appliqué de manière étanche sur ledit orifice de gonflage proximal (44) afin d'assurer l'étanchéité au fluide desdits ballonnets et de pouvoir être retiré dudit orifice de gonflage proximal (44) de manière à permettre l'évacuation du fluide desdits ballonnets.

10. Dispositif d'obstruction (70) selon la revendication 8, dans lequel ledit mécanisme de dégonflage comprend un ballonnet de dégonflage proximal pouvant être perforé (72) en communication fluidique avec ledit ballonnet distal (14).

11. Dispositif d'obstruction (70) selon la revendication 10, dans lequel ledit ballonnet de dégonflage (72) est de taille inférieure à celle dudit ballonnet distal (14).

12. Dispositif d'obstruction (70) selon la revendication 10, dans lequel ledit dispositif d'administration (90) comprend un outil d'insertion distal (92) qui comporte un arbre (100) servant à produire des déplacements grossier et fin dudit tube de gonflage (28).
